# EUROPÄISCHE PATENTANMELDUNG

(11) **EP 2 730 232 A1**
(43) Veröffentlichungstag der Anmeldung: **14.05.2014**
(21) Anmeldenummer: 13181976.5
(22) Anmeldetag: 28.08.2013
(51) Int. Cl.: A61B 17/04, A61B 19/00, A61B 17/06

(54) **Chirurgische Fadenspannvorrichtung**

(30) Priorität: 13.11.2012 DE 102012220602
(71) Anmelder: Mathys AG Bettlach, 2544 Bettlach (CH)
(72) Erfinder: Delfosse, Daniel, Dr., 3303 Jegenstorf (CH); Gyssler, Bernhard, 8800 Thalwil (CH); Gross, Christoph, 3027 Bern (CH)
(74) Vertreter: Körfer, Thomas

(57) **Zusammenfassung**

Chirurgische Fadenspannvorrichtung (10) zum Spannen eines an einem Implantat zu fixierenden Fadens mit einem rohrförmigen Schaft (11) einer axialen Durchgangsöffnung (18) und mit einem distalen Ende (22) und einem proximalen Ende (21). Die Fadenspannvorrichtung (10) hat ferner ein Kontaktelement (13), das mit dem proximalen Ende (21) des Schafts (11) verbunden ist und eine Öffnung (9) aufweist, die sich von dem dem Schaft (11) abgewandten Ende des Kontaktelements (13) in axialer Richtung teilweise durch das Kontaktelement (13) erstreckt, und ein Spannelement (14) mit einem Fadenhalter (15) zum Fixieren eines Fadens. Der Fadenhalter (15) ist dabei seitlich neben dem Schaft (11) angebracht, sodass der Faden außerhalb der axialen Durchgangsöffnung (18) verläuft und die axiale Durchgangsöffnung (18) zum Einbringen eines zusätzlichen Werkzeugs frei bleibt.

## Beschreibung

Die Erfindung bezieht sich auf eine chirurgische Fadenspannvorrichtung zum Spannen eines an einem Implantat zu fixierenden Fadens.

In chirurgischen Verfahren werden Fäden, die entweder aus einer einzelnen Faser oder einem Geflecht aus einer Vielzahl von Fasern bestehen, für eine Vielzahl von Anwendungen verwendet. Beispielsweise wird ein Faden zur Unterstützung eines gerissenen Kreuzbandes in ein Kniegelenk eingesetzt. Der Faden wir z.B. durch ein erstes Halteelement an einem ersten Knochen fixiert und mit einer vorbestimmten Spannung mit Hilfe eines Verankerungsimplantats, z.B. einer speziell geformten Knochenschraube, an einem zweiten Knochen befestigt, um die Zugbelastung auf das natürliche, zusammenwachsende Band während der Heilung zu reduzieren. Der Faden ist dabei federnd in dem Verankerungsimplantat fixiert, um die Zugbelastung auf den Faden zu reduzieren und gleichzeitig eine Bewegung des Kniegelenks zu ermöglichen, die dem physiologischen Bewegungsablauf nahe kommt.

Aus der US 2008/0275477 A1 ist eine Fadenspannvorrichtung bekannt, die ein kanüliertes Röhrchen, einen daran angeordneten Griff mit einem Schlitz zur Durchführung eines Fadens und eine in den Schlitz des Griffes reichende Fadenhalterung umfasst. Der zu spannende Faden wird in das kanülierte Röhrchen eingeführt und weiter durch den Griff zum Fadenhalter geführt und dort fixiert. Durch Drehen der Einstellvorrichtung wird anschließend der Faden gespannt.

Ist der Faden mit der gewünschten Spannkraft gespannt, muss dieser an einem Verankerungsimplantat im Knochen fixiert werden. Da die Fixierung bei gespanntem Faden vorgenommen werden muss, ist der Zugang zum Verankerungsimplantat oftmals durch die Fadenspannvorrichtung verdeckt bzw. nur durch Verrücken der Fadenspannvorrichtung und einer eventuellen Änderung der am Faden wirkenden Spannung zugänglich.

Es ist daher die Aufgabe der Erfindung, eine chirurgische Fadenspanneinrichtung zu schaffen, die neben dem Spannen des Fadens einen einfachen und bequemen Zugang zum Verankerungsimplantat im Knochen ermöglicht, um beispielsweise den Faden im Verankerungsimplantat in exakt der eingestellten Spannung zu fixieren.

Die Aufgabe wird durch die erfindungsgemäße chirurgische Fadenspannvorrichtung mit den Merkmalen des Anspruchs 1 gelöst. Die erfindungsgemäße chirurgische Fadenspannvorrichtung zum Spannen eines an einem Implantat, z.B. einem Verankerungsimplantat, zu fixierenden Fadens umfasst einen rohrförmigen Schaft mit einer axialen Durchgangsöffnung und einem distalen und einem proximalen Ende und einen Spannelement mit einem Fadenhalter zum Fixieren des Fadens, wobei der Fadenhalter nun seitlich neben dem Griff angebracht ist.

Bevorzugt ist ein Kontaktelement, das mit dem proximalen Ende des Schafts verbunden ist und eine Öffnung aufweist, die sich von dem dem Schaft abgewandten Ende des Kontaktelements in axialer Richtung zumindest teilweise durch das Kontaktelement erstreckt, vorhanden.

Ist die Fadenspannvorrichtung mit dem Kontaktelement am Implantat angesetzt, wird über die Öffnung im Kontaktelement der Faden aufgenommen und außerhalb des rohrförmigen Schafts zu dem seitlichen neben dem Schaft angebrachten Fadenhalter geführt und dort fixiert. Durch Drehen des Spannelements wird der Faden gespannt. Ein Werkzeug zum Fixieren des Fadens am Implantat kann nun durch die axiale Durchgangsöffnung im rohrförmigen Schaft zum Implantat geführt werden und dort den Faden z.B. durch Eindrehen einer Klemmschraube fixieren. Das Werkzeug kann somit unter Beibehaltung der Position der Fadenspannvorrichtung an das Implantat herangeführt werden. Eine Änderung der Fadenspannung durch ein aus seiner ursprünglichen Lage versetzte Fadenspannvorrichtung wird somit minimiert bzw. gänzlich ausgeschlossen.

Vorteilhafterweise weist die Außenkontur des Kontaktelements eine mehrkantige, insbesondere sechskantige, Kontur auf. Dies ist insbesondere vorteilhaft, wenn auch das Verankerungsimplantat eine Mehrkantinnenkontur aufweist. Die Fadenspannvorrichtung ist somit unbeweglich bezüglich einer Drehbewegung um ihre Längsachse als auch unbeweglich in Bezug auf eine axiale Bewegung zum bzw. weg vom Knochen, die durch eine Anschlagseite im Verankerungsimplantat gegeben ist, am Verankerungsimplantat fixiert. Somit ist ein Verrutschen oder Verdrehen der Spannvorrichtung während des Spannens ausgeschlossen. Dies garantiert das Beibehalten der eingestellten Zugspannung und stellt wird dadurch sicher, dass die gewünschte Fadenspannung auch am Fixierungspunkt im Verankerungselement selbst vorliegt. Zusätzlich werden eventuelle Verletzungen bei einem Verrutschen vermieden.

In einem vorteilhaften Ausführungsbeispiel ist das distale Ende des Schafts mit einem Griff verbunden und eine auf beiden Seiten offene in Längsrichtung verlaufende Durchgangsöffnung im Schaft und im Griff ausgebildet. Der Griff weist einen in axialer Richtung verlaufenden Schlitz auf, der von einem Ansatzbereich des Schaftes schräg in Richtung auf dem Fadenhalter verläuft. Der Griff ermöglicht ein bequemes Halten der Fadenspannvorrichtung ohne den Faden zwischen Hand und Griff einzuklemmen und somit den Spannvorgang zu behindern. Durch die von Griff und Schaft gemeinsam gebildete Durchgangsöffnung bleibt ein Kanal zur Durchführung von Werkzeugen erhalten.

Es ist ebenfalls von Vorteil, wenn das Spannelement an einem im Griff in Längsrichtung beweglich gelagerten Halteelement fixiert ist und eine Feder in einem Hohlraum im Griff angeordnet ist, die sich zwischen einer Anlagefläche des Halteelements und einer Anlagefläche im Griff abstützt. Das Halteelement weist vorteilhafterweise eine Skala auf, die einer vorbestimmten Spannkraft des Fadens entspricht. Dadurch ist die eingestellte Spannung des Fadens bequem ablesbar und durch Arretieren des Spannelements fixierbar.

Dies ist insbesondere mit der Sechskantaußenkontur des Kontaktelements von Vorteil, da durch den festen Sitz der Fadenspannvorrichtung im Verankerungsimplantat der Faden bei der eingestellten Spannung gehalten wird und gleichzeitig z.B. der Tibiaknochen eines behandelten Kniegelenks bewegt werden kann und somit die eingestellte Fadenspannung noch während der Operation überprüft werden kann. Vorteilhaft ist es dabei, wenn die Feder eine geringere Federkonstante als eine Feder im Verankerungsimplantat aufweist. Dies vergrößert den Federweg bei einer gegebenen Zugkraft in der Fadenspannvorrichtung gegenüber dem Federweg der Feder im Verankerungsimplantat und erlaubt somit eine präzise Einstellung einer gewünschten Spannkraft.

Weiterhin ist es von Vorteil, wenn der Schaft und/oder der Griff eine Öffnung aufweist bzw. aufweisen, die in umfänglicher Richtung weniger als 180 Grad geöffnet ist. Die Öffnung reicht in bevorzugter Weise in radiale Richtung bis zur Durchgangsöffnung. Dadurch kann der Verlauf des Fadens sowie das Einführen des zusätzlichen Werkzeugs in Fadenspannvorrichtung visuell verfolgt und überprüft werden. Somit können Unregelmäßigkeiten zeitnah erkannt und verhindert werden.

Von Vorteil ist es, wenn der Fadenhalter in Form einer Drehklemme ausgebildet ist. Eine Drehklemme weist z.B. eine oder zwei Backen auf, die jeweils um eine Achse drehbar sind und eine Bewegung des Fadens in Richtung der Zugspannung ermöglichen, eine Bewegung des Fadens in die entgegengesetzte Richtung aber verhindern. Somit kann der Faden einfach in eine Drehklemme eingeführt und ein Verlieren des Fadens bei Zug in die Gegenrichtung verhindert werden.

Die Drehklemme ist vorteilhafterweise lösbar mit dem Halteelement verbunden. Ebenso weist das Spannelement eine sattelförmige Halterung auf, die lösbar mit dem Halteelement verbunden ist. Dies ermöglicht eine einfache Montage und Reinigung der Fadenspannvorrichtung, da das Spannelement als Ganzes einfach vom Halteelement abmontiert werden kann.

Das Spannelement selbst umfasst zusätzlich eine Spanneinheit, die eine Fadenspule mit dem Fadenhalter und eine Sperre mit einem Drehgriff umfasst. Der Fadenhalter bildet vorteilhafterweise zumindest einen Teil einer Achse der Fadenspule aus. Somit kann der im Fadenhalter fixierte Faden durch Drehen des Drehgriffs auf die Achse der Fadenspule aufgewickelt werden. Die Sperre ermöglicht es, den Faden mit der eingestellten Spannung zu halten, ohne dass ein Operateur den Drehgriff festhalten muss. Dies ermöglicht in einfacher Weise die bereits erwähnte Kontrolle der Fadenspannung am z.B. operierten Gelenk ohne die Fadenspannvorrichtung aktiv in der eingestellten Spannung halten zu müssen.

Ausführungsbeispiele der erfindungsgemäßen chirurgischen Fadenspannvorrichtung sind in den Zeichnungen beispielhaft dargestellt und werden anhand der nachfolgenden Beschreibung näher erläutert. Es zeigen:
- Fig. 1: ein erstes Ausführungsbeispiel einer erfindungsgemäße Fadenspannvorrichtung mit Griff in perspektivischer Ansicht;
- Fig. 2: ein zweites Ausführungsbeispiel einer erfindungsgemäßen Fadenspannvorrichtung als Schnittansicht durch eine Längsachse;
- Fig. 3: ein drittes Ausführungsbeispiel einer erfindungsgemäßen Fadenspannvorrichtung mit einem Spannungsmesser als Schnittansicht durch eine Längsachse;
- Fig. 4: das dritte Ausführungsbeispiel einer erfindungsgemäßen Fadenspannvorrichtung in Draufsicht auf eine Öffnung in einem Griff bzw. Schaft;
- Fig. 5: ein erfindungsgemäßes Ausführungsbeispiel einer sattelförmigen Halterung einer erfindungsgemäßen Fadenspanneinrichtung in Draufsicht und
- Fig. 6: ein erfindungsgemäßes Ausführungsbeispiel einer Spanneinheit in Draufsicht.

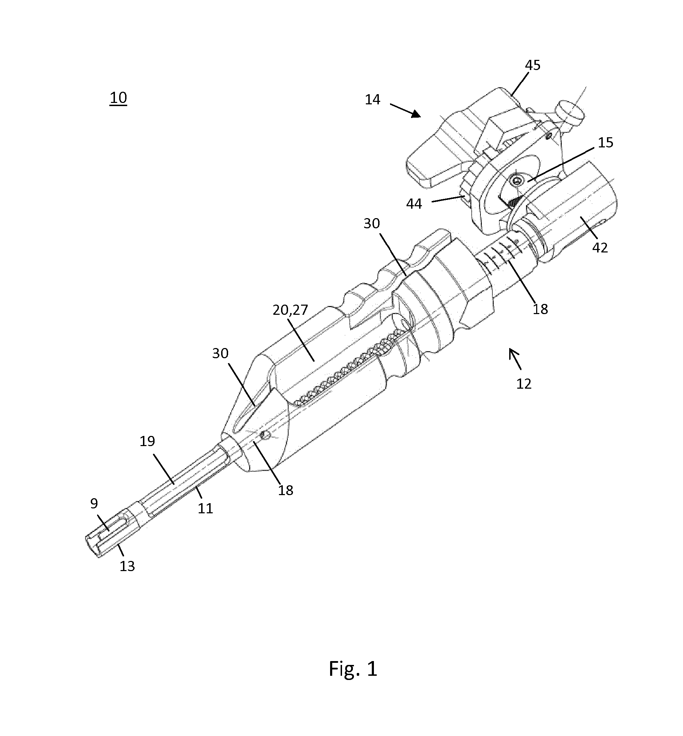

Einander entsprechende Teile sind in allen Figuren mit den gleichen Bezugszeichen versehen. Anhand von Fig. 1 wird nun der generelle Aufbau der Fadenspannvorrichtung erläutert und der Fadenverlauf beschrieben.

Die Fadenspannvorrichtung weist in einem Ende ein Koppelelement 13 auf. Das Koppel- oder Kontaktelement 13 ist rohrförmig mit einer Durchgangsöffnung, die sich in axialer Richtung durch das gesamte Koppelelement 13 erstreckt, ausgebildet. Das Koppelelement 13 weist eine Öffnung 9 auf, die sich von dem dem Knochen oder Implantat zugewandten Ende in axialer Richtung teilweise durch das Koppelelement erstreckt und in radialer Richtung bis zur Durchgangsöffnung reicht.

Das Koppelelement 13 ist mit einem rohrförmig ausgebildeten Schaft 11 verbunden. Der Schaft 11 weist ebenfalls eine in axialer Richtung, auch als Längsrichtung bezeichnet, durch die gesamte Länge des Schafts 11 verlaufende Durchgangsöffnung auf. Ein Spannelement 14 mit einem Fadenhalter 15 ist seitlich neben dem Schaft 11 angebracht. Der Fadenhalter 15 ist mit einem Gesperre 44 und einem Drehgriff 45 verbunden. Ein zu fixierender Faden, der hier der Übersicht halber nicht dargestellt ist, wird über die Öffnung 9 aus dem Koppelelement 13 herausgeführt und außerhalb des Schaftes 11 schräg in axiale Richtung zum Fadenhalter 15 geführt und dort fixiert. Durch Drehen des Fadenhalters 15 bzw. des Drehgriffs 45, der über ein Gesperre 44 mit dem Fadenhalter 15 verbunden ist, wird über eine Drehbewegung der Faden auf den Fadenhalter 15 aufgewickelt und damit gespannt.

Das rohrförmige Koppelelement 13 und der ebenfalls rohrförmige Schaft 11 bilden dabei einen Einführkanal in Form einer Durchgangsöffnung 18 für ein zusätzliches Werkzeug, das beispielsweise zum Fixieren des Fadens an einem Implantat benötigt wird. Ein solches Implantat ist beispielsweise ein Verankerungselement, das zur Halterung eines Fadens in einen Knochen implantiert ist. Der Faden wird außerhalb dieses Kanals geführt, sodass sich Faden und Werkzeug nicht gegenseitig beeinflussen und insbesondere nicht stören können.

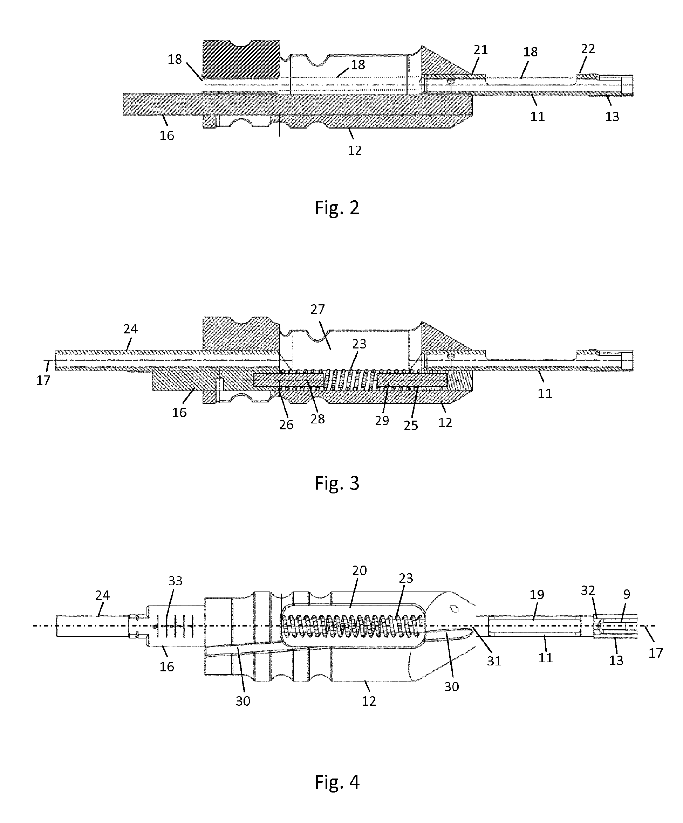

In dem abgebildeten Ausführungsbeispiel ist ein Griff 12 an dem dem Koppelelement 13 gegenüber liegenden distalen Ende 21 des Schafts 11 angeordnet. Der Griff 12 weist ebenfalls eine in axialer Richtung durch die gesamte Länge des Griffs 12 führende Durchführungsöffnung auf. Somit erstreckt sich eine Durchführungsöffnung 18 vom Koppelelement 13 über den Schaft 11 und den Griff durch die gesamte Länge der Fadenspannvorrichtung 10. Der Griff 12 ist bevorzugt aus Kunststoff gefertigt und weist einen Schlitz 30 auf, der in axialer Richtung schräg durch den Griff 12 verläuft. Der Schlitz 30 dient zur Aufnahme des Fadens und führt von einem Ansatzbereich 31 des Schaftes 11, siehe auch Fig. 4, in axialer Richtung schräg zum gegenüberliegenden Ende des Griffs 12 und tritt dort seitlich zur Achse 17 der Fadenspannvorrichtung 10 versetzt in Richtung auf den Fadenhalter aus, sodass der Faden gerade vom Koppelelement 13 zum Fadenhalter 15 verläuft.

Der Schaft 11 sowie optional auch der Griff 12 weisen eine Öffnung 19 bzw. 20 auf, die in umfänglicher Richtung einen Winkel von weniger als 180 Grad einnimmt und bis zur Durchführungsöffnung 18 reicht. Die Öffnung 19 bzw. 20 erlaubt somit einen direkten Blickkontakt in die Durchgangsöffnung 18. Dadurch kann ein Operateur den Fadenverlauf bzw. das Einführen des zusätzlichen Werkzeugs kontinuierlich beobachten und kontrollieren. Diese Durchgangsöffnung 18 erstreckt sich optional des Weiteren durch zusätzliche in axialer Richtung am Griff angeordnete Komponenten, wie z.B. ein Halteelement 16 und/oder eine Halterung 42, über die das Spannelement 14 am Griff 12 befestigt ist.

Fig. 2 zeigt ein zweites Ausführungsbeispiel der chirurgischen Fadenspannvorrichtung als Schnittdarstellung in axialer Richtung. Darin ist deutlich die Durchgangsöffnung 18 zu erkennen, die vom Koppelelement 13 durch den Schaft 11 und den Griff 12 hindurch ausgebildet ist. Hier ist auch dargestellt, dass der Schaft 11 mit einem distalen Ende 21 im Griff 12 fixiert ist. Am proximalen Ende 22 des Schafts 11 ist das Koppelelement 13 angeordnet. Das Koppelelement 13 kann einstückig mit dem Schaft 11 oder als separate Komponente ausgebildet sein. Ein Halteelement 16 bildet einen Bestandteil des Griffs 12 und dient insbesondere zur Befestigung des Spannelements 14 am Griff 12 bzw. in Verlängerung am Schaft 11.

Fig. 3 zeigt ebenfalls einen axialen Schnitt durch die in Fig. 1 dargestellte Fadenspannvorrichtung 10. In diesem Ausführungsbeispiel ist das Halteelement 16 mit einem rohrförmigen Element 24 fest verbunden. Das rohrförmige Element 24 kann auch einstückig mit dem Halteelement 16 ausgebildet sein. Der hohle Innenraum des rohrförmigen Elements 24 bildet eine Weiterführung der Durchgangsöffnung 18, siehe Fig. 2, aus.

In einem Hohlraum 27 des Griffs 12 ist eine Feder 23 eingebracht, die sich an einer Anlagefläche 26 des Halteelements 16 und eine Anlagefläche 25 im Griff 12 abstützt. Über die Anlagefläche 26 ragt ein erster Führungsstift 28 hervor. In gleicher Weise ragt ein zweiter Führungsstift 29 über die Anlagefläche 25 im Griff 12 hervor. Die Feder 23 übergreift den ersten und zweiten Führungsstift. Über den Abstand zwischen den zueinander gerichteten Enden des ersten und zweiten Führungsstiftes 28, 29 kann ein maximaler Kompressionsweg der Feder 23 und somit eine maximale Spannung des Fadens vorgegeben werden. Der erste und zweite Führungsstift 28 und 29 greifen jeweils in die z.B. als Spiralfeder ausgebildete Feder 23 ein und verhindern somit ein Verlieren oder Ausspringen der Feder 23 aus dem Griff 12.

Das Spannelement 14 ist dabei, wie in Fig. 1 dargestellt, am Halteelement 16 bzw. 24 unbeweglich in axialer Richtung fixiert. Wird nun der Faden durch Drehen des Drehgriffs 45 gespannt, wird das Halteelement 16 und das rohrförmige Element 24 entsprechend der wirkenden Kraft und Federkonstante der Feder 23 zum Schaft 11 hin verschoben. Über eine Skala 33, siehe Fig. 4, die am Halteelement 16 angebracht ist, kann bequem die wirkende Spannkraft abgelesen werden. Feder 23 und Skala 33 wirken somit als Spannungsmesser. Vorteilhaft ist es dabei, wenn die Feder 23 eine geringere Federkonstante als eine Feder im Verankerungsimplantat aufweist. Dies vergrößert den Federweg bei einer gegebenen Zugkraft in der Fadenspannvorrichtung gegenüber dem Federweg der Feder im Verankerungsimplantat und erlaubt somit eine präzise Einstellung einer gewünschten Spannkraft.

Fig. 4 zeigt das Ausführungsbeispiel der Fadenspannvorrichtung aus Fig. 3 bzw. Fig. 1 in Draufsicht. Dabei ist deutlich die Öffnung 20 im Griff 12 sowie die Öffnung 19 im Schaft 11 und die Öffnung 9 im Koppelelement 13 sichtbar. Die Achse 17 bildet hier eine gemeinsame Achse der Durchgangsöffnung 18 vom Koppelelement 13 bis zum rohrförmigen Element 24 aus.

Das Koppelelement 13 weist eine sechskantige Außenkontur 32 auf. Ebenfalls ist der Schlitz 30, der in axialer Richtung vom Schaft-seitigen Ende 31 des Griffs 11 bis zum gegenüberliegenden Ende des Griffs 12 verläuft, deutlich zu sehen. Der Schlitz 30 verläuft dabei zur Achse 17 geneigt, sodass der Faden sich im geradlinigen Verlauf durch den Griff 12 hindurch zur nicht abgebildeten Fadenhalterung 15 erstreckt.

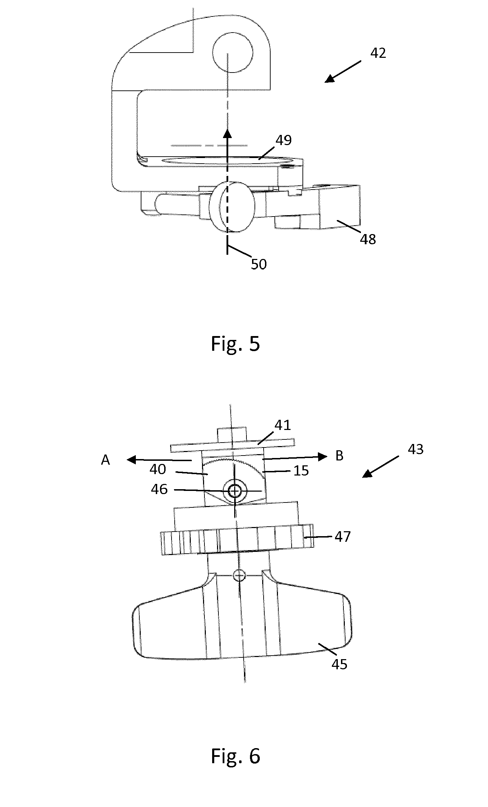

Fig. 5 zeigt die sattelförmige Halterung 42 der Spannvorrichtung 14, mit der die Spannvorrichtung 14 am Schaft 11 bzw. am Halteelement 16 oder rohrförmigen Element 24 lösbar befestigt ist. In eine Einschuböffnung 49 wird in Pfeilrichtung 50 eine Spanneinheit 43 eingeschoben und lösbar fixiert.

Die Spanneinheit 43, siehe Fig. 6, umfasst eine Fadenspule 41, deren Achse zumindest teilweise von dem Fadenhalter 15 gebildet wird. Fest mit der Fadenspule 41 sind ein Sperrrad 47 sowie der Drehgriff 45 verbunden. Befindet sich die Spanneinheit 43 in der sattelförmigen Halterung 42, so liegt die Sperrklinke 48 auf dem Sperrrad 47 auf und erlaubt in Spannrichtung das Drehen des Sperrrads 45. In entgegengesetzter Richtung greift die Sperrklinke 48 in einen Zacken des Sperrrads ein und blockiert die Drehung der Spanneinheit 43.

Der Fadenhalter 15 selber ist hier beispielsweise durch eine einbackige Drehklemme 40 ausgebildet. Diese Drehklemme 40 ist drehbar um die Achse 46 gelagert. Ein Faden kann dabei ohne signifikanten Widerstand in Pfeilrichtung A durch die Fadenhalterung gezogen werden. Bei einem Zug auf den Faden in die entgegengesetzte Richtung wird die Drehklemme 40 an die seitliche Wand der Fadenspule 41 gepresst und klemmt somit den Faden ein. Somit ist der Faden insbesondere unter einer Spannkraft in Pfeilrichtung B fixiert.

Durch den modularen Aufbau des Spannelements 14 mit der sattelförmigen Halterung 42 und die darin arretierbare Spanneinheit 43 kann die Fadenspannvorrichtung einfach gereinigt oder auch gewartet werden.

Alle beschriebenen und/oder gezeigten Merkmale können im Rahmen der Erfindung vorteilhaft miteinander kombiniert werden. Die Erfindung ist nicht auf die beschriebenen Ausführungsbeispiele beschränkt.

## Patentansprüche

1. Chirurgische Fadenspannvorrichtung zum Spannen eines an einem Implantat zu fixierenden Fadens mit einem rohrförmigen Schaft (11) mit einer axialen Durchgangsöffnung (18), einem distalen Ende (22) und einem proximalen Ende (21), und
einem Spannelement (14) mit einem Fadenhalter (15) zum Fixieren eines Fadens,
**dadurch gekennzeichnet,**
**dass** der Fadenhalter (15) seitlich neben dem Schaft (11) angebracht ist.

2. Vorrichtung nach Anspruch 1
**gekennzeichnet durch**,
ein Koppelelement (13), das mit dem proximalen Ende (21) des Schafts (11) verbunden ist und eine Öffnung (9) aufweist, die sich von dem dem Schaft (11) abgewandten Ende des Koppelelements (13) in axialer Richtung zumindest teilweise **durch** das Koppelelement (13) erstreckt.

3. Vorrichtung nach Anspruch 2,
**dadurch gekennzeichnet,**
**dass** die Außenkontur (32) des Koppelelements (13) eine mehrkantige, insbesondere 6-kantige, Kontur aufweist.

4. Vorrichtung nach einem der Ansprüche 1 bis 3,
**dadurch gekennzeichnet,**
**dass** ein Griff (12) mit dem distale Ende (22) des Schafts (11) verbunden ist und eine auf beiden Seiten offene in Längsrichtung verlaufende Durchgangsöffnung (18) durch den Schaft (11) und den Griff (12) ausbildet ist.

5. Vorrichtung nach Anspruch 4,
**dadurch gekennzeichnet,**
**dass** der Griff (12) einen in Längsrichtung verlaufenden Schlitz (30) aufweist, der von einem Ansatzbereich (31) des Schaftes (11) schräg in Richtung auf den Fadenhalter (15) bis zum gegenüberliegenden Ende des Griffs (12) verläuft.

6. Vorrichtung nach Anspruch 4 oder 5,
**dadurch gekennzeichnet,**
**dass** das Spannelement (14) an einem im Griff (12) in axiale Richtung beweglich gelagerten Halteelement (16) fixiert ist,
**dass** eine Feder (23) in einem Hohlraum (27) im Griff (12) angeordnet ist, die sich zwischen einer Anlagefläche (26) des Halteelement (16) und einer Anlagefläche (25) im Griff (12) abstützt und
**dass** das Halteelement (16) eine Skala (33) aufweist, deren Skalierung einer vorbestimmten Spannkraft des Fadens entspricht.

7. Vorrichtung nach einem der Ansprüche 1 bis 6,
**dadurch gekennzeichnet,**
**dass** der Schaft (11) eine Öffnung (19) aufweist, die bis zur Durchgangsöffnung (18) reicht und in umfänglicher Richtung weniger als 180° geöffnet ist.

8. Vorrichtung nach einem der Ansprüche 4 bis 6,
**dadurch gekennzeichnet,**
**dass** der Griff (12) eine Öffnung (20) aufweist, die bis zur Durchgangsöffnung (18) reicht und in umfänglicher Richtung weniger als 180° geöffnet ist.

9. Vorrichtung nach Anspruch 7 oder 8,
**dadurch gekennzeichnet,**
**dass** die Öffnung (19, 20) im Schaft (11) bzw. im Griff (12) sich in axialer Richtung jeweils nicht bis zum Ende des Schafts (11) bzw. Griffs (12) erstreckt.

10. Vorrichtung nach einem der Ansprüche 1 bis 9,
**dadurch gekennzeichnet,**
**dass** der Fadenhalter (15) in Form einer Drehklemme (40) ausgebildet ist.

11. Vorrichtung nach Anspruch 10,
**dadurch gekennzeichnet,**
**dass** die Drehklemme (15) lösbar mit dem Halteelement (16) verbunden ist.

12. Vorrichtung nach Anspruch 6,
**dadurch gekennzeichnet,**
**dass** das Spannelement (14) eine sattelförmige Halterung (42) aufweist, die lösbar mit dem Halteelement (16) verbunden ist.

13. Vorrichtung nach Anspruch 12,
**dadurch gekennzeichnet,**
**dass** das Spannelement (14) eine Spanneinheit (43) aufweist, die lösbar mit der sattelförmigen Halterung (42) verbunden ist, und
**dass** die Spanneinheit (43) eine Fadenspule (41) mit Fadenhalter (15) und ein Gesperre (44) mit einem Drehgriff (45) aufweist.

14. Vorrichtung nach Anspruch 13,
**dadurch gekennzeichnet,**
**dass** der Fadenhalter (15) zumindest einen Teil einer Achse der Fadenspule (41) bildet.
